# EUROPÄISCHE PATENTSCHRIFT

(11) **EP 0 416 572 B1**
(45) Veröffentlichungstag und Bekanntmachung des Hinweises auf die Patenterteilung: **17.05.1995**
(21) Anmeldenummer: 90117061.3
(22) Anmeldetag: 05.09.1990
(51) Int. Cl.: C12N 15/82, C12N 15/11, C07H 21/02, A01H 5/00, A01H 5/10

(54) **RNA mit Endoribonucleaseaktivität gegen mRNA von Reifungsgenen, ihre Herstellung und ihre Verwendung in Pflanzen**
RNAs with endoribonucleasic activity against mRNA from ripening genes, their preparation and their use in plants
RNAs à activité endoribonucléasique contre le mRNA de gènes de maturation, leur préparation et leur utilisation dans des plantes

(30) Priorität: 07.09.1989 DE 3929741
(43) Veröffentlichungstag der Anmeldung: 13.03.1991
(73) Patentinhaber: Hoechst Schering AgrEvo GmbH, 13342 Berlin (DE)
(72) Erfinder: Uijtewaal, Bernardus, Dr., NL-6093 JG Heythheuysen (NL); Schneider, Rudolf, Dr., D-6233 Kelkheim/Taunus (DE); Uhlmann, Eugen, Dr., D-6246 Glashütten/Taunus (DE); Müllner, Hubert, Dr., D-6233 Kelkheim/Taunus (DE)

(56) Entgegenhaltungen:
- EP-A- 0 271 988
- UCLA SYMPOSIA ON MOLECULAR AND CELLULAR BIOLOGY NEW SERIES, Vol. 129. PLANTGENE TRANSFER; SYMPOSIUM, PARK CITY, UTAH, USA, APRIL 1-7, 1989 THEOLOGIS, A.et al.:"Cloning the messenger RNA of ACC synthase."
- NATURE. vol. 334, 21 Juli 1988, LONDON GB Seiten 196-197; WALBOT, V. & BRUENING, G.: Plant development and ribozymes for pathogens".
- NATURE. vol. 334, 18 August 1988, LONDON GB; GERLACH,W.: "Simple RNA enzymes with new and highly specific endoribonuclease activities." S. 585-591

## Beschreibung

RNA-Moleküle können unter geeigneten Voraussetzungen ohne Beteiligung von Proteinen an anderen RNA-Molekülen Reaktionen katalysieren oder autokatalytisch Fragmente aus eigenen Molekülen abspalten. So wird vom 3′-Ende der 23s rRNA von Tetrahymena thermophila autokatalytisch ein Intron mit 413 Nukleotiden entfernt und in eine zirkuläre Form übergeführt. Dies geschieht durch eine Reihe von Phosphoestertransfer-Reaktionen mit Beteiligung von Guanosin-Kofaktoren (Cech, T.R., Nature 30, 578-583 (1983)). Je nach RNA Substrat oder den gewählten Reaktionsbedingungen kann das Intron als spezifische Ribonuclease, terminale Transferase, Phosphotransferase oder saure Phosphatase funktionieren. Dabei kann ein RNA-Molekül einige Umsetzungen vornehmen, ohne selbst verändert zu werden und verhält sich in dieser Hinsicht wie ein Enzym. Für RNA-Moleküle mit diesen Eigenschaften wurde deshalb der Begriff Ribozym geprägt.

Ähnliche Reaktionen ohne Beteiligung von Proteinen konnten auch für einige viroide RNAs und Satelliten-RNAs gezeigt werden. So scheint für Avocado Sunblotch Viroid (ASBV) (Hutchins, C.J. et al. Nucleic Acids Res. 14, 3627-3640 (1986)), Satelliten-RNA von Tobacco Ringspot Virus (sTobRV) (Prody, G.A. et al, Science 231, 1577-1580 (1986)) und Satelliten-RNA von Luzerne Transient Streak Virus (sLTSV) (Forster A.C. et al., Cell 49, 211-220 (1987)) eine Selbst-Prozessierung eine essentielle Reaktion für die Vermehrung zu sein. Während der Replikation dieser RNAs werden vermutlich zirkuläre Formen gebildet, die als "Templates" zur Synthese von RNAs mit Überlänge führen. Diese Transkripte werden durch die selbstkatalysierten, endonucleolytischen Reaktionen auf die richtige Genomlänge zurechtgeschnitten.

Die Strukturen der RNAs, die diese vermutlich für die Reaktion einnehmen, wurden als "hammerheads" beschrieben (Forster A. C. et al., Cell 49, 211-220 (1987); Haseloff, J. et al., Nature 334, 585-591 (1988)).

Die Schnittstellen für diese RNA-Enzyme sind spezifisch und müssen bestimmte strukturelle Voraussetzungen aufweisen, damit eine Prozessierung erfolgen kann.

Es wurde nun gefunden, daß Ribozyme für Reifungsenzyme kodierende Pflanzen-RNA angreifen können und damit zur Beeinflussung der Reifungsprozesse in Pflanzen eingesetzt werden können.

Die Regulation der Expression der für das Reifungsenzym Polygalakturonase kodierenden DNA durch "antisense"-RNA wurde von Smith C.J.S. et al. in Nature 334, 724 (1988) beschrieben. Ein Fragment der Polygalakturonase-cDNA wird in gegensinniger Orientierung in einen Expressions-Vektor gebracht. Mit diesem Vektorplasmid werden über E. coli und Agrobacterium tumefaciens Stiel-Segmente der Tomate transformiert. Die Expression von antisense-RNA kann dann in den Blättern der Tomatenpflanze nachgewiesen werden. Man nimmt an, daß die "antisense"-RNA sich an die eigentliche Polygalakturonase-RNA anlagert, wodurch diese inaktiviert wird, was wiederum zur Folge hat, daß die Polygalakturonase-Synthese zum Teil inhibiert wird.

Zur spezifischen Beeinflussung der Reifungsprozesse in Pflanzen wurden nun Ribozyme entwickelt, die an Reifungsenzym-RNA binden und diese an bestimmten Schnittstellen in der Sequenz spalten können. Mit Hilfe der erfindungsgemäßen Ribozyme kann die Synthese bestimmter Reifungsenzyme nicht nur zum Teil inhibiert, sondern nahezu vollständig, i.e. ca. 80-100 %, gehemmt werden.

Die Erfindung betrifft somit:
1. Ribozyme kodierende Gene bzw. Gen-Fragmente, sowie die entsprechenden Ribozym RNA-Sequenzen gemäß der Ansprüche 1-3.
2. Pflanzen, Pflanzenzellen sowie Teile oder Samen der Pflanzen, die die unter 1. genannte DNA- oder RNA-Sequenz enthalten.
3. Die Verwendung von Ribozymen zur Hemmung der Synthese von Reifungsenzymen in Pflanzen.

Im folgenden wird die Erfindung detailliert beschrieben, insbesondere in ihren bevorzugten Ausführungsformen. Ferner wird die Erfindung in den Ansprüchen definiert.

Das Ribozym kann auf Basis der DNA-Sequenz des zu hemmenden Reifungsgens synthetisiert werden. Dabei kann grundsätzlich von jeder für ein pflanzliches Reifungsenzym kodierenden DNA-Sequenz ausgegangen werden. Derartige pflanzliche Reifungsenzyme sind beispielsweise Polygalakturonase, Pektinesterase und sogenannte "ripening related proteins". Bevorzugt werden als "Vorlage" für die Synthese mindestens 10 aufeinanderfolgende Nukleotide, insbesondere 14 bis 20 Nukleotide, vorteilhaft aus der Mitte der cDNA-Sequenz des Strukturgens ausgewählt. Es ist insbesondere vorteilhaft, jeweils von der cDNA-Sequenz für Polygalakturonase (Grierson, D. et al., NAR 14, 8595 (1986)), Pektinesterase (Ray, J. et al., Eur. J. Biochem. 174, 119 (1988)) sowie für das "ripening related protein" (Ray, J. et al., NAR 15, 10587 (1987)) auszugehen.

Auf Basis der cDNA-Sequenz werden Oligonukleotide chemisch so synthetisiert, daß die jeweils aus mindestens 5 Nukleotiden, vorteilhaft 7 bis 10 Nukleotiden bestehenden Anfangs- bzw. Endsequenzen komplementär zu der DNA, bzw. Nukleotiden bestehen, die zusammengenommen komplemenar zu einer DNA-Sequenz des zu hemmenden Reifungsenzyms sind und daß die Anfangs- und Endsequenz der Oligonukleotide durch eine dazwischenliegende RNA-Sequenz getrennt werden, die zum Teil aus spezifischen, für die Funktionalität des Ribozyms vorgegebenen Nukleotiden und zum Teil aus variablen Nukleotiden besteht. Das mit Substrat-RNA hybridisierte Ribozym kann im Schema wie folgt aussehen. 
5-NNNNNNNNNNNNNGUC NNNNNNNNNNNNNNN-3 ← Substrat-RNA
3-KKKKKKKKCA KKKKKKK-5
A C
A U
G G
G A A
C G G V
V V
V V
VL VL
VL VL
VL VL
Loop
Ribozym
wobei
- N: Nukleotide der pflanzlichen Reifungsenzym-RNA, A, C, G oder U
- K: zur pflanzlichen Reinfungsenzym-RNA komplementäre Nukleotide A, C, G, oder U im Ribozym
- V: variable Nukleotide A, C, G oder U im Ribozym und
- V_{L}: variable Nukleotide A, C, G oder U im Loop des Ribozyms bedeutet.
Die Nukleotidanzahl von V_{L} im Loop kann 0-550 betragen. Als Schnittstelle in der Substrat-RNA wird bevorzugt eine GU-Erkennungssequenz gewählt.

Die genannten Oligonukleotide werden mit einem entsprechenden Linker versehen. Derartige Linker besitzen beispielsweise Schnittstellen von EcoRI, SalI, BamHI, HindIII, EcoRV, SmaI, XhoI, KpnI, bevorzugt XbaI bzw. PstI.

Die synthetisierten Oligonukleotide werden mit Hilfe der Vektoren pUC19, pUC 18 oder pBluescript (Stratagene, Heidelberg, Product Information) kloniert, und sequenziert.

Das bestätigte Oligonukleotid wird in einen intermediären Vektor mit Pflanzenpromotor kloniert. Derartige Vektoren sind beispeilsweise die Plasmide pPCV701 (Velten J. et al. EMBO J. 3, 2723-2730 (1984)), pNCN (Fromm M. et al. PNAS 82, 5824-5826 (1985)) oder pNOS ( An G. et al., EMBO J. 4, 277-276 (1985)). Bevorzugt wird der Vektor pDH51 (Pietrzak, M. et al., Nucleic Acids Res. 14, 5857, (1986)) mit einem 35S-Promotor verwendet.

Nach anschließender Transformation von E. coli, wie z.B. E. coli MC 1061, DH1, DK1, GM48 oder XL-1, werden positive Klone nach an sich bekannten Methoden (Maniatis et al., Lab. Manual), wie Plasmidminipräparation und Spaltung mit einem entsprechenden Restriktionsenzym, identifiziert.

Diese positiven Klone werden dann in einen binären Pflanzenvektor subkloniert. Als Pflanzenvektoren können pGV3850 (Zambrysk, P. et al, EMBO J. 2, 2143-2150 (1983)) oder pOCA18 (Olszewski, N., Nucleic Acids Res. 16, 10765-10782, (1988)) eingesetzt werden. Vorteilhaft wird mit pOCA18 gearbeitet.

Die erhaltenen binären Pflanzenvektoren, die einen Pflanzenpromotor mit dem angehängten DNA-Fragment für die Ribozym-Produktion in der T-DNA enthalten, werden verwendet, um Pflanzen zu transformieren. Dies kann durch Techniken wie Elektroporation oder Mikroinjektion erfolgen.

Bevorzugt wird die Kokultivierung von Protoplasten oder die Transformation von Blattstückchen mit Agrobakterien angewandt. Dazu wird das Pflanzenvektorkonstrukt durch Transformation mit gereinigter DNA oder, vermittelt über einen Helferstamm wie E. coli SM10 (Simon R. et al., Biotechnology 1, 784-791 (1983)), in Agrobakterium tumefaciens wie A282 mit einem Ti Plasmid über ein "triparental mating" transferiert.

Direkte Transformation und triparental mating wurden, wie in, "Plant Molecular Biology Manual" (Kluwer Academic Pubishers, Dordrecht (1988)) beschrieben, durchgeführt.

Es können grundsätzlich alle Pflanzen mit Ribozym-DNA tragenden binären Pflanzenvektoren transformiert werden. Bevorzugt sind dikotyledone Pflanzen, insbesondere Nutzpflanzen, wie z.B. fruchttragende Pflanzen. Als Beispiel sollen Tomate, Erdbeere, Avocado, sowie Pflanzen, die tropische Früchte tragen, z.B. Papaya, Mango, aber auch Birne, Apfel, Nektarine, Aprikose oder Pfirsich genannt werden. Insbesondere bevorzugt wird das beschriebene Verfahren mit der Tomate durchgeführt. Die transformierten Zellen werden mit Hilfe eines Selektionsmediums selektiert, zu einem Kallus herangezüchtet und auf einem entsprechenden Medium zur Pflanze regeneriert (Shain et al, Theor. appl. Genet. 72, 770-770 (1986); Masson, J. et al., Plant Science 53, 167-176 (1987), Zhan et al., Plant Mol. Biol. 11, 551-559 (1988); McGranaham et al, Bio/Technology 6, 800-804 (1988). Novrate et al., Bio/Technol. 7, 154-159 (1989)).

Die resultierende Pflanze wird durch die Transformation insofern verändert, als in den Zellen die Ribozyme exprimiert werden, was wiederum bewirkt, daß die Ribozym-RNA nicht nur an die den entsprechenden Reifungsgenen komplementäre RNA bindet und eine mehr oder weniger starke Hemmung der Synthese des Reifungsenzyms bewirkt, sondern daß die den entsprechenden Reifungsgenen komplementäre RNA spezifisch an GUC-Sequenzen geschnitten wird, wodurch die Synthese des betreffenden Reifungsenzyms nahezu vollständig gehemmt wird.

Die Bildung der Ribozym-spezifischen Sekundärstrukturmerkmale der in der transgenen Pflanze in vivo synthetisierten Ribozym-RNA war in keiner Weise zu erwarten, so daß die beobachtete Hemmung der Synthese der Reifungsenzyme völlig überraschend war.

Die folgenden Beispiele dienen der weiteren Erläuterung der Erfindung.

### Beispiele

Prozentangaben beziehen sich auf das Gewicht, soweit nichts anderes angegeben ist.

### 1. Klonierung der Oligonukleotide

Die Synthese der Oligonukleotide zur Ribozymexpression erfolgte auf Basis der folgenden cDNA-Sequenzen 
a) 5' TGATGGAGTCCATGTATCA 3' | Ausschnitt aus der
Polygalakt. cDNA-Sequenz
nach Grierson, D. et al.,
Nucleic Acids Res. 14,
8595-8603

b) 5' TAGCAAGTCCTGACCTAA 3' | Ausschnitt aus der
cDNA-Sequenz der Pektin-
Esterase nach Ray, J.,
Eur. J. Biochem. 174,
119-124 (1988)

c) 5' TGCTTTGTCCGATACAGT 3' | Sequenzausschnitt der
cDNA eines "ripening
related protein"
nach Ray, J., Nucleic
Acids Res. 15, 10587
(1987)
Mit Hilfe der Phosphoramidit-Methode (Engels J. et al., Advances in Biochemical Engineering Biotechnology Band 37 ed.: A. Fiechter, Springer Verlag, Berlin/Heidelberg, 1988) wurden folgende Oligonukleotide mit einem Synthesizer synthetisiert: 
zu a) 5' -CTAGATGATACATGCTGATGAGTCCGTGAGGACGAAACTCCATCTGCA 3'
3'TACTATGTACGACTACTCAGGCACTCCTGCTTTGAGGTAG 5'

zu b) 5' -CTAGATTAGGTCAGCTGATGAGTCCGTGAGGACGAAACTTGCTACTGCA-3'
3'-TAATCCAGTCGACTACTCAGGCACTCCTGCTTTGAACGATG-5'

zu c) 5'-CTAGACTGTATCGCTGATGAGTCCGTGAGGACGAAACAAAGCACTGCA-3'
3'-TGACATAGCGACTACTCAGGCACTCCTGCTTTGTTTCGTG-5'
Der Vektor pDH51 (Pietrzak, M. et al, Nucleic Acids Res. 14, 5857 19) wurde mit den Restriktionsendonukleasen XbaI und PstI geschnitten, mit "calf intestinal phosphatase" (CIP) inkubiert, phenolisiert und gefällt (Maniatis, Lab. Manual). Der so behandelte Vektor wurde mit einem dreifachen Überschuß an phosphorylierten Oligonukleotiden ligiert und in E. coli MC1061 transformiert. Positive Klone wurden durch Plasmidminipräparationen und anschließende Verdauung mit XbaI und PstI identifiziert.

Ferner wurden die ampicillin-resistenten transformierten E. coli Zellen (100 »g Ampicillin pro ml LB-Medium) auf Nitrocellulose-Membranen (Gene Screen Plus®, NEN®, Boston) überführt und weitere 14 Stunden bei 37°C auf LB-medium mit Ampicillin inkubiert. Die Kolonien wurden danach in 0,5 M NaOH aufgeschlossen und fixiert. Nach dem Trocknen konnten die Filter mit radioaktiv markierten Oligonukleotiden hybridisiert werden. Positive Klone ergaben eine Schwärzung auf dem Film.

### 2. Subklonierung eines 35S-Promotor-Genfragments in pOCA 18

Aus den nach 1. erhaltenen Klonen wurde je ein 0,75 kb EcoRI Fragment isoliert. Dieses wurde in einen mit EcoRI geschnittenen pOCA 18 Vektor eingebaut und in E. coli MC1061 transformiert. Positive Klone wurden durch Plasmidminipräparationen und nach anschließender Hydrolyse mit EcoRI durch das Auftreten der 0,75 kb-Bande identifiziert.

### 3. Transformation von Agrobacterium tumefaciens

Um Pflanzen transformieren zu können, muß das unter 2. erhaltene Konstrukt in ein Agrobakterium überführt werden. Dies geschieht entweder durch "triparental mating" oder direkt. Beim "triparental mating" wurden je 100 »l Bakterien aus Übernachtkulturen von E. coli SM10, der die Konstruktion tragenden E. coli MC1061 und Agrobacterium tumefaciens abzentrifugiert und zusammen in 30 »l LB Medium aufgenommen. Nach 30 Minuten bei Raumtemperatur gab man diese Bakteriensuspension auf einem Filter auf eine LB Platte ohne Antibiotika. Nach 12 Std. Inkubation bei 37°C wurde der Filter in 2,5 ml 10 mM MgCl₂ gewaschen. Aliquots wurden auf LB Platten mit Rifampicin, Tetrazyklin und Kanamycin selektiert. Positive Kolonien wurden durch Hybridisierung mit ³²P markierter DNA des zu exprimierenden Gens identifiziert.

Bei der direkten Transformation von Agrobakterien wurden die Zellen über Nacht in YEB Medium (1 % Yeast-Extrakt, 1 % Pepton, 0,5 % NaCl) mit 25 »g/ml Kanamycin und 100 »g/ml Rifampicin bei 28°C gezüchtet. Die Bakteriensuspension wurde nach 16 Stunden auf eine OD₅₅₀ von 0,1 verdünnt und bis zu einer OD₅₅₀ von 0,5 bei 28°C weiterinkubiert. 1 ml dieser Kultur wurde abzentrifugiert und mit 1 ml 150 mM NaCl gewaschen. Nach dem Waschen resuspendierte man den Niederschlag in 600 »l eiskalter 10 mM CaCl₂-Lösung.

Aus nach 2. gewonnene E. coli Klone wurde nach Aufschluß der Zellen mit 0,2 N NaOH/ 1 % SDS der pOCA 18-Vektor mit dem klonierten 0,75 kb-Fragment isoliert und über eine CsCl-Dichtegradientenzentrifugation gereinigt. 1 »g der Plasmid-DNA gab man zu den kompetenten Agrobakterien und stellte die Eppendorf-Gefäße für 1 Stunde auf Eis. Nach 1 Stunde inkubierte man die Plasmid-Lösung 5 Minuten bei 37°C und gab 2 ml YEB Medium dazu. Die Zellen wurden anschließend über Nacht bei 28°C in einem Schüttelinkubator inkubiert. Danach gab man jeweils 100 »l auf YEB-Platten mit 100 »g/ml Rifampicin, 25 »g/ml Kanamycin und 2,5 »g/ml Tetrazyklin. Nach 2 Tagen bei 28°C zeigten sich auf den Platten Kolonien. Positive Klone wurden durch Hybridisierung mit den entsprechenden ³²P markierten Oligonukleotiden a), b) oder c) nachgewiesen.

Dazu wurden die transformierten Agrobakterien auf Gene Screen Plus Membranen ausgestrichen und auf YEB-Platten mit 100 »g/ml Rifampicin, 25 »g/ml Kanamycin mit 2,5 »g/ml Tetrazyklin bei 28°C 14 Stunden inkubiert. Die Membranen wurden anschließend 2 Minuten auf 0,5 M NaOH und danach 2 Minuten auf 0,5 M Tris pH 7,5 gelegt. Nach dem Trocknen prähybridisierte man in 10 % Dextransulfat/ 1 M NaCl/ 1 % SDS bei 55°C für 2 Stunden und gab danach die radioaktiv markierte Oligonukleotide a), b) oder c) dazu. Die Filter wurden über Nacht bei 55°C zusammen mit den radioaktiv markierten Oligos inkubiert. Nach dem Waschen bei 55°C für je 30 Minuten mit 1x SSC (0,15 M NaCl, 0,015 M Natriumcitrat, pH 7,0) und 2x mit 0,2 fachen SSC wurden die positiven Klone durch Schwärzung eines aufgelegten Films identifiziert.

### 4. Transformation von Tomaten

a) Protoplastentransformation:
   Tomatenprotoplasten (Plant Cell Reports 6, 172-175 (1987)) werden einmal mit W5-Lösung (154 mM NaCl, 125 mM CaCl₂ · H₂O, 5 mM KCl, 5 mM Glukose) und einmal mit MaMg-Lösung (0,45 M Mannitol, 25 mM MgCl₂, 0,1 % 2-(N-Morpholin)ethansulfonsäure (MES, pH 5,8), Fa. Sigma Chemie, Deisenhofen, BR Deutschland) gespült. Nach vorsichtigem Zentrifugieren bei 600 rpm über 3 Minuten wird der Überstand bis auf 0,5 ml abgezogen. Dazu pipettiert man 50 »g Kalbsthymus DNA, 10 »g des beschriebenen Plasmids und 10 Tropfen 45 % Polyethylenglykol (PEG 8000). Nach 10 Minuten werden die Protoplasten zweimal mit W5-Lösung gewaschen und in LCM-Medium (Plant Cell Reports 6, 172-175 (1987)) inkubiert.
b) Blattstückchentransformation mit Agrobakterien:
   Tomatenblätter werden kleingeschnitten und auf MS-Medium (Murashige, T. et al., Physiol. Plant 15, 473-497 (1962)), mit 2 % Saccharose und 1 ppm Pflanzenwachstumshormon Zeatin (Serva Feinbiochemica GmbH & Co., Heidelberg, BR Deutschland) gelegt und bei 25°C über einen Zeitraum von 16 Stunden Licht/Tag inkubiert. Einen Tag später erfolgt Infektion mit Agrobakterien. Dabei werden die Blattstückchen kurz in eine verdünnte Bakteriensuspension (OD 0,15) des transformierten Agrobakterienstamms eingetaucht, auf dieselbe Platte zurückgelegt und unter gleichen Bedingungen weiterinkubiert.
   Am dritten Tag werden alle Blattstückchen mit einer 250 mg/l Carbenicillinlösung gespült und auf ein 2MS-Medium mit 1 ppm Zeatin, 200 mg/l Cefotaxim (Hoechst AG, Frankfurt), 200 mg/l Carbenicillin und 100 mg/l Kanamycin, gegeben. Regeneranten wurden nach etwa 20 weiteren Tagen auf 2 MS-medium mit 1 ppm Zeatin, 200 mg/l Cefotaxim, 200 mg/l Carbenicillin und 100 mg/l Kanamycin umgesetzt.

Nachweis der gegen die "ripening related protein" -RNA gerichteten Ribozym-RNA einer transgenen Tomatenpflanze.

Aus einem Blattstückchen einer transgenen Tomatenpflanze, die mit dem gegen die "ripening related protein"-RNA gerichteten Ribozym-kodierenden Gen transformiert wurde, wurde die zelluläre Gesamt-RNA isoliert. Dazu zerrieb man das Blattmaterial unter flüssigem Stickstoff mit Mörser und Pistill. Zum pulverförmigen Blattmaterial gab man das doppelte Volumen eines Extraktionspuffers (0,2 M Natriumacetat, 1 % SDS, 10 mM EDTA), das doppelte Volumen Phenol (equilibriert mit Extraktionspuffer) und ein halbes Volumen Chloroform/Isoamylalkohol 24:1 (v/v). Nach sehr gründlichem Vermischen trennte man die Phasen durch Zentrifugieren und wiederholte die phenolische Extraktion der oberen wäßrigen Phase. Diese wäßrige Phase wurde nach Durchmischen und Trennen durch zentrifugieren wieder in ein frisches Eppendorf-Gefäß überführt und mit Chloroform/Isoamylalkohol 24:1 (v/v) extrahiert. Danach gab man zur wäßrigen Phase 1/3 Volumen 8 M LiCl und hielt den Ansatz über Nacht bei 4°C. Nach dem Zentrifugieren bildete sich ein Niederschlag. Dieser wurde in Wasser aufgenommen und nach Zugabe von 0,3 M Na-Azetat (pH 5,5) (Endkonzentration) mit 2 1/2 Volumen Ethanol gewaschen. Nach dem Waschen mit 70 % Ethanol und Trocknen wurde der Niederschlag in 50 »l Wasser aufgenommen.

Zum Nachweis der spezifischen Expression des Ribozymcodierenden Gens wurden je etwa 4 »g Gesamt-RNA aus einer nicht-transformierten Wildtyptomatenpflanze und aus einer transgenen Tomatenpflanze auf einem 1 % Agarosegel mit 6 % Formaldehyd aufgetragen. Zum Auftragen wurde die RNA getrocknet, in 50 % Formamid/6 % Formaldehyd aufgenommen und für 15 Minuten auf 60°C erhitzt. Das Agarosegel wurde nach dem Lauf kurz mit H₂O gewaschen und mit 10 x SSC auf Gene Screen Plus Membran übertragen. Die Membran wurde nach 24 Stunden mit 2 x SSC gespült, 2 Std. bei 80°C inkubiert und getrocknet.

Die Hybridisierung erfolgte nach 2 Stunden Prähybridisierung mit 1 % SDS, 1 M NaCl und 10 % Dextransulfat bei 55°C mit radioaktiv markiertem Oligonukleotid c) des "ripening related protein"-DNA.

Nachweis des gegen die "ripening related protein"-RNA gerichteten ribozymcodierenden Gens

2 Blattstückchen der transgenen Tomatenpflanze bzw. einer nicht-transformierten Wildtyptomate wurden unter flüssigem Stickstoff mit Mörser und Pistill zerkleinert. Das Pulver wurde in Eppendorfgefäße gegeben und mit 500 »l 2 x CTAB-Puffer versetzt (2 x CTAB: 2 % Cetyl-Trimethylammoniumbromid, 100 mM Tris pH 8,0, 20 mM EDTA, 1,4 M NaCl, 1 % Polyvinylpyrrolidon MG = 40000), der vorher auf 65°C erhitzt wurde. Anschließend gab man 500 »l Chloroform/Isoamylalkohol 24:1 (v/v) dazu und extrahierte die wäßrige Phase. Die beiden Phasen wurden anschließend durch zentrifugation getrennt. Die wäßrige Phase pipettierte man in ein neues Eppendorfgefäß und gab 100 »l 5 % CTAB dazu, das auf 65°C erhitzt wurde. Es wurde nochmal mit Chloroform/Isoamylalkohol extrahiert, bevor 500 »l CTAB-Präzipitierungspuffer (1 % CTAB, 50 mM Tris pH 8,0, 10 mM EDTA) zur wäßrigen Phase zugegeben wurde. Nach Zentrifugation wurde der Niederschlag in Hochsalz TE (10 mM Tris pH 8,0, 1 mM EDTA, 1 M NaCl) gelöst und mit 2 1/2 Volumen Ethanol gefällt. Nach Zentrifugation, Waschen und Trocknen nahm man den Niederschlag in Wasser auf und behandelte mit 25 »g/ml RNase A (Endkonzentration). Die RNase wurde anschließend durch Phenolisieren entfernt. Nach erneutem Trocknen nahm man die DNA in 50 »l Wasser auf.

Anschließend wurden 4 »g der DNA 1 Stunde bei 37°C mit der Restriktionsendonuklease Eco RI hydrolysiert. Das Hydrolysat wurde auf einem 1 % Agarosegel aufgetrennt. Das Gel schüttelte man 30 Min. mit 0,4 N NaOH/0,6 M NaCl und anschließend 30 Min. mit 0,5 M Tris Cl pH 7,5/1,5 M NaCl. Als Größenstandard diente ein PstI-Hydrolysat der λ-Phagen-DNA.

Anschließend transferierte man die DNA auf ein Gene Screen Plus Membran mit 10 x SSC über einen Kapillar-Blot. Das Filter wurde anschließend getrocknet und mit 1 % SDS/1 M NaCl/ 10 % Dextransulfat prähybridisiert. Zum Hybridisieren versetzte man den Prähybridisierungsmix mit einer radioaktiv markierten Probe des Oligonukleotides c), das vorher 10 Minuten gekocht wurde.

Das Ribozym-codierende Gen wurde durch das Auftreten einer Schwärzung des aufgelegten Röntgenfilms bei ca. 0,8 kb nachgewiesen.

### Nachweis der in vitro Aktivität der Ribozyme

Das Oligonukleotid das ein Ribozym gegen die "ripening related protein"-RNA codierte wurde in den nach Hydrolyse mit den Restriktionsendonukleasen XbaI und PstI geöffneten Vektor Bluescript kloniert. Parallel dazu wurde in denselben Vektor ein DNA-Fragment des "ripening related proteins" (Nukleotidnummer 792-815 der von Ray, J. et al., Nucleic Acids Res. 15, 10587 (1987) veröffentlichten DNA-Sequenz) in die SacI/KpnI Schnittstelle kloniert. Beide Vektoren dienten in einer in vitro RNA Polymerase Reaktion als DNA-Matrize für die RNA-Synthese.

Hierzu wurden parallel der das Ribozym-Gen-tragende Vektor und der das DNA-Fragment des "ripening related protein"-Gen-tragende Vektor mit der Restriktionsendonuklease SacI geschnitten. Je 1 »g der geöffneten Vektoren wurden anschließend mit je 10 »M der Nukleotide ATP, GTP, CTP, UTP und (α-³²P)UTP (5 ...) in 50 mM HEPES (pH 7,5) und 10 u. T7 RNA-Polymerase für 30 Minuten bei 37°C inkubiert. Die RNA wurde nach DNase Behandlung in Ethanol gefällt.

Die synthetisierte RNA des Ribozym-Gen-tragenden Vektors und die RNA des "ripening related protein"-Genfragmenttragenden Vektors wurden zusammen in 50 mM Tris·Cl (pH 7,8) und 10 mM MgCl₂ zwei Stunden bei 25°C inkubiert. Die Reaktionsprodukte wurden anschließend auf einem 5 %igen denaturierenden Polyacrylamidgel (8 M Harnstoff) getrennt und durch Autoradiographie auf einem Röntgenfilm identifiziert. Das Autoradiogramm zeigt, daß in Anwesenheit von Ribozym-RNA das RNA-Transkript des "ripening related protein"-Genfragmentes gespalten wurde.

### Nachweis der verzögerten Reifung von transgenen Tomaten

Ein Vergleich einer nicht-transformierten Wildtyp-Tomatenpflanze mit einer transgenen Tomatenpflanze, die das gegen die "ripening related protein"-RNA gerichtete Ribozym-kodierende Gen trägt, ergab, daß die Tomaten der transgenen Pflanze eine um mehrere Tage verzögerte Reifung zeigten. Die Ribozym-Aktivität und die damit verbundene Reifungsverzögerung wurde somit auch in der Tomatenfrucht, die den eigentlichen Wirkort darstellt, nachgewiesen.

## Patentansprüche (Patentansprüche für folgende(n) Vertragsstaat(en): AT, BE, CH, DE, DK, FR, GB, IT, LI, NL, SE)

1. Ribozym kodierendes Gen oder Gen-Fragment mit der DNA-Sequenz

a) 5' CTAGATGATACATGCTGATGAGTCCGTGAGGACGAAACTCCATCTGCA 3'
3'TACTATGTACGACTACTCAGGCACTCCTGCTTTGAGGTAG 5'

b) 5' -CTAGATTAGGTCAGCTGATGAGTCCGTGAGGACGAAACTTGCTACTGCA-3'
3'-TAATCCAGTCGACTACTCAGGCACTCCTGCTTTGAACGATG-5'

c) 5'-CTAGACTGTATCGCTGATGAGTCCGTGAGGACGAAACAAAGCACTGCA-3'
3'-TGACATAGCGACTACTCAGGCACTCCTGCTTTGTTTCGTG-5'

2. RNA mit Ribozym-Aktivität der Sequenz, 
3-KKKKKKKKCA   KKKKKKKK-5
A   C
A     U
G   A   G
            A
C G   G
            V
V V
V V
V V
V       V   Loop
V V
Ribozym
 in der
K zur pflanzlichen Reifungsenzym-RNA komplementäre Nukleotide
A, C, G oder U,
V variable Nukleotide A, C, G oder U und
V_{L} variablen Nukleotide A, C, G oder U im Loop bedeutet, wobei die Nukleotidanzahl von V_{L} im Loop eine Zahl von 0 bis 550 ist.

3. RNA mit Ribozym-Aktivität mit der Sequenz

a)        C U A C C U C A     G U A C A U A G U
                                    C
                          A
                                    U
                          A
                                    G
                          G         A
                          C  G A G U
                          A  U
                          G  C
                          G  C
                         A    U
                           C
                          G

b)        A U C G U U C A     G A C U G G A U U
                                    C
                          A
                                    U
                          A
                                    G
                          G         A
                          C  G A G U
                          A  U
                          G  C
                          G  C
                         A    U
                           C
                          G

c)        A C G A A A C A     G C U A U G U C A
                                    C
                          A
                                    U
                          A
                                    G
                          G
                                    A
                          C  G A G U
                          A  U
                          G  C
                          G  C
                         A    G
                           U
                          G

4. Verfahren zur Herstellung eines Ribozym kodierenden Gens oder Gen-Fragments mit der DNA-Sequenz 
a) 5' CTAGATGATACATGCTGATGAGTCCGTGAGGACGAAACTCCATCTGCA 3'
3'TACTATGTACGACTACTCAGGCACTCCTGCTTTGAGGTAG 5'

b) 5' -CTAGATTAGGTCAGCTGATGAGTCCGTGAGGACGAAACTTGCTACTGCA-3'
3'-TAATCCAGTCGACTACTCAGGCACTCCTGCTTTGAACGATG-5'

c) 5'-CTAGACTGTATCGCTGATGAGTCCGTGAGGACGAAACAAAGCACTGCA-3'
3'-TGACATAGCGACTACTCAGGCACTCCTGCTTTGTTTCGTG-5'
 durch Synthese von Oligonukleotiden, dadurch gekennzeichnet, daß Oligonucleotide synthetisiert werden, deren Anfangs- und Endsequenzen aus jeweils mindestens 5, vorzugsweise 7 bis 10 Nukleotiden bestehen, die zusammengenommen komplementär zu einer DNA-Sequenz des zu hemmenden pflanzlichen Reifungsenzyms sind und durch eine dazwischenliegende DNA-Sequenz getrennt werden, die zum Teil aus spezifischen, für die Funktionalität des Ribozyms vorgegebenen Nukleotiden und zum Teil aus variablen Nukleotiden besteht.

5. Verfahren zur Herstellung von RNA mit Ribozym-Aktivität der Sequenz, 
3-KKKKKKKKCA   KKKKKKK-5
A   C
A     U
G   A     G
              A
C G   G   V
V V
V V
VL VL
VL      VL   Loop
VL VL
Riboz
 dadurch gekennzeichnet, daß ein Oligonukleotid der Sequenz 
5'-K'K'K'K'K'K'K'K'CTGAVGAG

3'-K K K K K K K K GACTVCTC

V'V'VL'VL'VL'VL'VL'VLV'VCGAAACK'K'K'K'K'K'K'-3'
V V VLVLVLVLVLVLV VGCTTTGK K K K K K K -5'
 in der
K zur pflanzlichen Reifungsenzym-RNA komplementäre Nukleotide A, C, G oder T,
V variable Nukleotide A, C, G oder T,
V_{L} variablen Nukleotide A, C, G oder T bedeutet, wobei die Nukleotidanzahl von V_{L} eine Zahl von 0 bis 550 ist und
K′, V′, V_{L}′ jeweils zu K, V, V_{L} komplementäre Nukleotide A, C, G oder T ist, synthetisiert wird,
welches in einen intermediären Vektor mit Pflanzenpromotor kloniert wird, dann zusammen mit dem Pflanzenpromotor in einen binären Pflanzenvektor kloniert wird und mit der so erhaltenen Plasmid-DNA eine Pflanze transformiert wird.

6. Verfahren nach Anspruch 5, dadurch gekennzeichnet, daß als RNA mit Ribozym-Aktivität eine RNA der Sequenz 
a)        C U A C C U C A     G U A C A U A G U
                                  C
                          A
                                  U
                          A
                                  G
                          G       A
                          C  G A G U
                          A  U
                          G  C
                          G  [?]
                        A    U

b)        A U C G U U C A     G A C U G G A U U
                                  C
                          A
                                  U
                          A
                                  G
                          G       A
                          C  G A G U
                          A  U
                          G  C
                          G  C
                        A    U
                           C
                          G
 
oder

c)      A C G A A A C A     G C U A U G U C A
                            C
                    A
                            U
                    A
                            G
                    G
                            A
                    C  G A G U
                    A  U
                    G  C
                    G  C
                   A   G
                     U
                    G
 synthetisiert wird.

7. Pflanzenzellen, Pflanzen, deren Samen und Teile, enthaltend eine oder mehrere der DNA-Sequenzen nach Anspruch 1.

8. Pflanzenzellen, Pflanzen, deren Samen und Teile, enthaltend eine oder mehrere der RNA-Sequenzen nach einem der Ansprüche 2 oder 3.

9. Tomate, Teile davon, deren Pflanzenzellen oder Samen, enthaltend eine oder mehrere der DNA-Sequenzen nach Anspruch 1.

10. Tomate, Teile davon, deren Pflanzenzellen oder Samen, enthaltend eine oder mehrere der RNA-Sequenzen nach einem der Ansprüche 2 oder 3.

11. Verwendung von RNA mit Ribozym-Aktivität gemäß Anspruch 2 zur Hemmung der Synthese von Reifungsenzymen in Pflanzen.

12. Verwendung nach Anspruch 11, dadurch gekennzeichnet, daß die Pflanzen fruchttragende Pflanzen sind.

13. Verwendung nach Anspruch 12, dadurch gekennzeichnet, daß die fruchttragenden Pflanzen Tomaten sind.

14. Verwendung nach einem oder mehreren der Ansprüche 11 bis 13, dadurch gekennzeichnet, daß als Ribozym die RNA-Sequenz nach Anspruch 3 eingesetzt wird.

## Patentansprüche (Patentansprüche für folgende(n) Vertragsstaat(en): ES)

1. Verfahren zur Herstellung eines Ribozym kodierenden Gens oder Gen-Fragments mit der DNA-Sequenz 
a) 5' CTAGATGATACATGCTGATGAGTCCGTGAGGACGAAACTCCATCTGCA 3'
3'TACTATGTACGACTACTCAGGCACTCCTGCTTTGAGGTAG 5'

b) 5' -CTAGATTAGGTCAGCTGATGAGTCCGTGAGGACGAAACTTGCTACTGCA-3'
3'-TAATCCAGTCGACTACTCAGGCACTCCTGCTTTGAACGATG-5'

c) 5'-CTAGACTGTATCGCTGATGAGTCCGTGAGGACGAAACAAAGCACTGCA-3'
3'-TGACATAGCGACTACTCAGGCACTCCTGCTTTGTTTCGTG-5'
 durch Synthese von Oligonukleotiden, dadurch gekennzeichnet, daß Oligonucleotide synthetisiert werden, deren Anfangs- und Endsequenzen aus jeweils mindestens 5, vorzugsweise 7 bis 10 Nukleotiden bestehen, die zusammengenommen komplementär zu einer DNA-Sequenz des zu hemmenden pflanzlichen Reifungsenzyms sind und durch eine dazwischenliegende DNA-Sequenz getrennt werden, die zum Teil aus spezifischen, für die Funktionalität des Ribozyms vorgegebenen Nukleotiden und zum Teil aus variablen Nukleotiden besteht.

2. Verfahren zur Herstellung von RNA mit Ribozym-Aktivität der Sequenz, 
3-KKKKKKKKCA   KKKKKKKK-5
A   C
A     U
G       G
   A       A
C G   G V
V V
V V
VL VL
V       VL   Loop
VL VL
Ribozym
 dadurch gekennzeichnet, daß ein Oligonukleotid der Sequenz 
5'-K'K'K'K'K'K'K'K'CTGAVGAG

3'-K K K K K K K K GACTVCTC

V'V'V_L'V_L'V_L'V_L'V_L'V_L'V'VCGAAACK'K'K'K'K'K'K'-3'
V V V_LV_LV_LV_LV_LV_LV VGCTTTGK K K K K K K -5'
 in der
K zur pflanzlichen Reifungsenzym-RNA komplementäre Nukleotide A, C, G oder T,
V variable Nukleotide A, C, G oder T,
V_{L} variablen Nukleotide A, C, G oder T bedeutet, wobei die Nukleotidanzahl von V_{L} eine Zahl von 0 bis 550 ist und
K′, V′, V_{L}′ jeweils zu K, V, V_{L} komplementäre Nukleotide A, C, G oder T ist, synthetisiert wird,
welches in einen intermediären Vektor mit Pflanzenpromotor kloniert wird, dann zusammen mit dem Pflanzenpromotor in einen binären Pflanzenvektor kloniert wird und mit der so erhaltenen Plasmid-DNA eine Pflanze transformiert wird.

3. Verfahren nach Anspruch 2, dadurch gekennzeichnet, daß als RNA mit Ribozym-Aktivität eine RNA der Sequenz 
a)    C U A C C U C A    G U A C A U A G U
                       C
                  A
                       U
                  A
                       G
                       A
                  G
                  C  G A G U
                  A  U
                  G  C
                  G  C
                 A   U
 
b)        A U C G U U C A    G A C U G G A U U
                           C
                   A
                           U
                   A
                           G
                   G       A
                   C  GAGU
                   A  U
                   G  C
                   G  C
                  A   U
                   GC

oder
c)        A C G A A A C A    G C U A U G U C A
                           C
                   A
                           U
                   A
                           G
                   G
                           A
                   C  GAGU
                   A  U
                   G  C
                   G  C
                  A   G
                   GU
 synthetisiert wird.

4. Verwendung von RNA mit Ribozym-Aktivität erhältlich nach Anspruch 2 zur Hemmung der Synthese von Reifungsenzymen in Pflanzen.

5. Verwendung nach Anspruch 4, dadurch gekennzeichnet, daß die Pflanzen fruchttragende Pflanzen sind.

6. Verwendung nach Anspruch 5, dadurch gekennzeichnet, daß die fruchttragenden Pflanzen Tomaten sind.

7. Verwendung nach einem oder mehreren der Ansprüche 4 bis 6, dadurch gekennzeichnet, daß als Ribozym die nach Anspruch 3 erhältliche RNA-Sequenz eingesetzt wird.

## Claims (Claims for the following Contracting State(s): AT, BE, CH, DE, DK, FR, GB, IT, LI, NL, SE)

1. A ribozyme-encoding gene or gene fragment having the DNA sequence

a)    5' CTAGATGATACATGCTGATGAGTCCGTGAGGACGAAACTCCATCTGCA 3'
          3'TACTATGTACGACTACTCAGGCACTCCTGCTTTGAGGTAG 5'

b)    5' -CTAGATTAGGTCAGCTGATGAGTCCGTGAGGACGAAACTTGCTACTGCA-3'
          3'-TAATCCAGTCGACTACTCAGGCACTCCTGCTTTGAACGATG-5'

c)    5'-CTAGACTGTATCGCTGATGAGTCCGTGAGGACGAAACAAAGCACTGCA-3'
          3'-TGACATAGCGACTACTCAGGCACTCCTGCTTTGTTTCGTG-5'

2. RNA with ribozyme activity of the sequence 
3-KKKKKKKKCA KKKKKKKK-5
A C
A U
G G
G A A
C G G A
V
V V
V V
V V
V V Loop
V V
ribozyme
 in which
K are nucleotides A, C, G or U complementary to the plant ripening enzyme RNA,
V are variable nucleotides A, C, G or U and
V_{L} are variable nucleotides A, C, G or U in the loop, where the number of V_{L} nucleotides in the loop is a number from 0 to 550.

3. RNA with ribozyme activity with the sequence

a)  C U A C C U C A   G U A C A U A G U
C
A
U
A
G
G
A
C  G A G U
A  U
G  C
G  C
A  U
C
G

b)  A U C G U U C A   G A C U G G A U U
C
A
U
A
G
G
A
C  G A G U
A  U
G  C
G  C
A  U
C
G

c)  A C G A A A C A   G C U A U G U C A
C
A
U
A
G
G
A
C  G A G U
A  U
G  C
G  C
A  G
U
G

4. A process for preparing a ribozyme-encoding gene or gene fragment having the DNA sequence 
a) 5' CTAGATGATACATGCTGATGAGTCCGTGAGGACGAAACTCCATCTGCA 3'
3'TACTATGTACGACTACTCAGGCACTCCTGCTTTGAGGTAG 5'

b) 5' -CTAGATTAGGTCAGCTGATGAGTCCGTGAGGACGAAACTTGCTACTGCA-3'
3'-TAATCCAGTCGACTACTCAGGCACTCCTGCTTTGAACGATG-5'

c) 5'-CTAGACTGTATCGCTGATGAGTCCGTGAGGACGAAACAAAGCACTGCA-3'
3'-TGACATAGCGACTACTCAGGCACTCCTGCTTTGTTTCGTG-5'
 by synthesis of oligonucleotides, which comprises synthesizing oligonucleotides whose initial and final sequences are each composed of at least 5, preferably 7 to 10, nucleotides which, taken together, are complementary to a DNA sequence of the plant ripening enzyme to be inhibited and are separated by an interpolated DNA sequence which is composed partly of specific nucleotides predetermined for the functionality of the ribozyme and partly of variable nucleotides.

5. A process for preparing RNA with ribozyme activity of the sequence 
3-KKKKKKKKCA  KKKKKKKK-5
A   C
A     U
G       G
G   A     A
C G   G
V
V V
V V
V V
V   V   Loop
V V
ribozyme
 which comprises synthesizing an oligonucleotide of the sequence 
5'-K'K'K'K'K'K'K'K'CTGAVGAG

3'-K K K K K K K K GACTVCTC

V'V'V_L'V_L'V_L'V_L'V_L'V_L'V'VCGAAACK'K'K'K'K'K'K'K'-3'
V V V_LV_LV_LV_LV_LV_LV VGCTTTGK K K K K K K K -5'
 in which
K are nucleotides A, C, G or T complementary to the plant ripening enzyme RNA,
V are variable nucleotides A, C, G or T,
V_{L} are variable nucleotides A, C, G or T, where the number of V_{L} nucleotides is a number from 0 to 550, and
K', V', V_{L}' is in each case nucleotides A, C, G or T complementary to K, V, V_{L},
which is cloned into an intermediary vector with plant promoter, then cloned together with the plant promoter into a binary plant vector, and a plant is transformed with the plasmid DNA obtained in this way.

6. The process as claimed in claim 5, wherein the synthesized RNA with ribozyme activity is an RNA of the sequence

a) C U A C C U C A   G U A C A U A G U
C
A
U
A   G
A
G
C GAGU
A U
G C
G C
A U

b)

A U C G U U C A    G A C U G G A U U
                   C
A                  U
A                  G
G                  A
C  GAGU
A  U
G  C
G  C
A  U
 GC

or

c)  A C G A A A C A    G C U A U G U C A
                       C
    A                  U
    A                  G
    G                  A
    C  GAGU
    A  U
    G  C
    G  C
    A  G
     GU

7. Plant cells, plants, the seeds and parts thereof, containing one or more of the DNA sequences as claimed in claim 1.

8. Plant cells, plants, the seeds and parts thereof, containing one or more of the RNA sequences as claimed in either of claims 2 or 3.

9. A tomato, parts thereof, plant cells or seeds thereof, containing one or more of the DNA sequences as claimed in claim 1.

10. A tomato, parts thereof, plant cells or seeds thereof, containing one or more of the RNA sequences as claimed in either of claims 2 or 3.

11. The use of RNA with ribozyme activity as claimed in claim 2 for inhibiting the synthesis of ripening enzymes in plants.

12. The use as claimed in claim 11, wherein the plants are fruit-bearing plants.

13. The use as claimed in claim 12, wherein the fruit-bearing plants are tomatoes.

14. The use as claimed in one or more of claims 11 to 13, wherein the RNA sequence as claimed in claim 3 is employed as ribozyme.

## Claims (Claims for the following Contracting State(s): ES)

1. A process for preparing a ribozyme-encoding gene or gene fragment having the DNA sequence 
a) 5' CTAGATGATACATGCTGATGAGTCCGTGAGGACGAAACTCCATCTGCA 3'
3'TACTATGTACGACTACTCAGGCACTCCTGCTTTGAGGTAG 5'

b) 5' -CTAGATTAGGTCAGCTGATGAGTCCGTGAGGACGAAACTTGCTACTGCA-3'
3'-TAATCCAGTCGACTACTCAGGCACTCCTGCTTTGAACGATG-5'

c) 5'-CTAGACTGTATCGCTGATGAGTCCGTGAGGACGAAACAAAGCACTGCA-3'
3'-TGACATAGCGACTACTCAGGCACTCCTGCTTTGTTTCGTG-5'
 by synthesis of oligonucleotides, which comprises synthesizing oligonucleotides whose initial and final sequences are each composed of at least 5, preferably 7 to 10, nucleotides which, taken together, are complementary to a DNA sequence of the plant ripening enzyme to be inhibited and are separated by an interpolated DNA sequence which is composed partly of specific nucleotides predetermined for the functionality of the ribozyme and partly of variable nucleotides.

2. A process for preparing RNA with ribozyme activity of the sequence 
3-KKKKKKKKCA  KKKKKKKK-5
A   C
A     U
G  A    G
C G  G   A
        V
V V
V V
V V
V    V      Loop
V V
ribozyme
 which comprises synthesizing an oligonucleotide of the sequence 
5'-K'K'K'K'K'K'K'K'CTGAVGAG

3'-K K K K K K K K GACTVCTC

V'V'V_L'V_L'V_L'V_L'V_L'V_L'V'VCGAAACK'K'K'K'K'K'K'K'-3'
V V V_LV_LV_LV_LV_LV_LV VGCTTTGK K K K K K K K -5'
 in which
K are nucleotides A, C, G or T complementary to the plant ripening enzyme RNA,
V are variable nucleotides A, C, G or T,
V_{L} are variable nucleotides A, C, G or T, where the number of V_{L} nucleotides is a number from 0 to 550, and
K', V', V_{L}' is in each case nucleotides A, C, G or T complementary to K, V, V_{L},
which is cloned into an intermediary vector with plant promoter, then cloned together with the plant promoter into a binary plant vector, and a plant is transformed with the plasmid DNA obtained in this way.

3. The process as claimed in claim 2, wherein the synthesized RNA with ribozyme activity is an RNA of the sequence

a)    C U A C C U C A    G U A C A U A G U
                                  C
                  A
                                  U
                  A
                                  G
                  G               A
                  C  G^A G^U
                  A  U
                  G  C
                  G  C
                A    U

b)    A U C G U U C A    G A C U G G A U U
                                  C
                  A
                                  U
                  A
                                  G
                  G               A
                  C  G^A G^U
                  A  U
                  G  C
                  G  C
                A    U
                   C
                  G

or

c)    A C G A A A C A    G C U A U G U C A
                                  C
                  A
                                  U
                  A
                                  G
                  G
                                  A
                  C  G^A G^U
                  A  U
                  G  C
                  G  C
                A    G
                   U
                  G

4. The use of RNA with ribozyme activity obtainable as claimed in claim 2 for inhibiting the synthesis of ripening enzymes in plants.

5. The use as claimed in claim 4, wherein the plants are fruit-bearing plants.

6. The use as claimed in claim 5, wherein the fruit-bearing plants are tomatoes.

7. The use as claimed in one or more of claims 4 to 6, wherein the RNA sequence obtainable as claimed in claim 3 is employed as ribozyme.

## Revendications (Revendications pour l'(les) Etat(s) contractant(s) suivant(s): AT, BE, CH, DE, DK, FR, GB, IT, LI, NL, SE)

1. Gène ou fragment de gène codant pour le ribozyme avec la séquence d'ADN

a) 5' CTAGATGATACATGCTGATGAGTCCGTGAGGACGAAACTCCATCTGCA 3'
3'TACTATGTACGACTACTCAGGCACTCCTGCTTTGAGGTAG 5'

b) 5' -CTAGATTAGGTCAGCTGATGAGTCCGTGAGGACGAAACTTGCTACTGCA-3'
3'-TAATCCAGTCGACTACTCAGGCACTCCTGCTTTGAACGATG-5'

c) 5'-CTAGACTGTATCGCTGATGAGTCCGTGAGGACGAAACAAAGCACTGCA-3'
3'-TGACATAGCGACTACTCAGGCACTCCTGCTTTGTTTCGTG-5'

2. ARN ayant une activité de ribozyme de la séquence, 
3-KKKKKKKKCA  KKKKKKK-5
A  C
A   U
    G
G A   A
C G G V
V V
V V
V V  Boucle
V V V V
Ribozyme
 dans laquelle
K représente les nucléotides A, C, G ou U complémentaires à l'ARN de l'enzyme de maturation végétale,
V représente les nucléotides variables A, C, G ou U, et
V_{L} représente les nucléotides variables A, C, G ou U dans la boucle, le nombre de nucléotides de V_{L} dans la boucle étant un nombre de 0 à 550.

3. ARN ayant une activité de ribozyme avec la séquence

a)    C U A C C U C A    G U A C A U A G U
                                C
              A
                                U
              A
                                G
              G                 A
              C  G^A G^U
              A  U
              G  C
              G  C
             A    U
               G^C

b)    A U C G U U C A    G A C U G G A U U
                                C
              A
                                U
              A
                                G
              G                 A
              C  G^A G^U
              A  U
              G  C
              G  C
             A    U
               G^C

c)    A C G A A A C A    G C U A U G U C A
                                C
              A
                                U
              A
                                G
              G
                                A
              C  G^A G^U
              A  U
              G  C
              G  C
             A    G
               G^U

4. Procédé pour la préparation d'un gène ou d'un fragment de gène codant pour un ribozyme ayant la séquence d'ADN 
a) 5' CTAGATGATACATGCTGATGAGTCCGTGAGGACGAAACTCCATCTGCA 3'
3'TACTATGTACGACTACTCAGGCACTCCTGCTTTGAGGTAG 5'

b) 5' -CTAGATTAGGTCAGCTGATGAGTCCGTGAGGACGAAACTTGCTACTGCA-3'
3'-TAATCCAGTCGACTACTCAGGCACTCCTGCTTTGAACGATG-5'

c) 5'-CTAGACTGTATCGCTGATGAGTCCGTGAGGACGAAACAAAGCACTGCA-3'
3'-TGACATAGCGACTACTCAGGCACTCCTGCTTTGTTTCGTG-5'
 par synthèse des oligonucléotides, caractérisé en ce qu'on synthétise des oligonucléotides dont les séquences initiale et terminale sont constituées chacune d'au moins 5, de préférence de 7 à 10 nucléotides qui ensemble sont complémentaires à une séquence d'ADN de l'enzyme de maturation végétale à inhiber et qui sont séparées par une séquence d'ADN intercalée entre les deux, qui est constituée en partie de nucléotides spécifiques prédéterminés pour la fonctionnalité du ribozyme et en partie de nucléotides variables.

5. Procédé pour la préparation d'ARN ayant une activité ribozymique de la séquence 
3-KKKKKKKKCA KKKKKKKK-5
A C
A U
G G
G A A
C G G V
V V
V V
V V
V V boucle
V V
Ribozyme
 caractérisé en ce qu'on synthétise un oligonucléotide de la séquence 
5'-K'K'K'K'K'K'K'K'CTGAVGAG

3'-K K K K K K K K GACTVCTC

V'V'V'LV'LV'LV'LV'LV'LV'VCGAAACK'K'K'K'K'K'K'-3'
V V VLVLVLVLVLVLV VGCTTTGK K K K K K K -5'
 dans laquelle
K représente les nucléotides A, C, G ou T complémentaires à l'ARN de l'enzyme de maturation végétale,
V représente les nucléotides variables A, C, G ou T, et
V_{L} représente les nucléotides variables A, C, G ou T, le nombre de nucléotides de V_{L} étant un nombre de 0 à 550.
K', V', V_{L}' , sont chaque fois les nucléotides A, C, G ou T complémentaires à K, V, V_{L}, lequel oligonucléotide est cloné dans un vecteur intermédiaire avec un promoteur végétal, puis, ensemble avec le promoteur végétal, il est cloné dans un vecteur végétal binaire et une plante est transformée par l'ADN plasmidique ainsi obtenue.

6. Procédé selon la revendication 5, caractérisé en ce qu'on synthétise en tant qu'ARN ayant une activité de ribozyme, un ARN de la séquence

a)        C U A C C U C A     G U A C A U A G U
                          C
                    A
                          U
                    A
                          G
                    G     A
                    C  GAGU
                    A  U
                    G  C
                    G  C
                    A  U

b)        A U C G U U C A     G A C U G G A U U
                          C
                    A
                          U
                    A
                          G
                    G     A
                    C  GAGU
                    A  U
                    G  C
                    G  C
                    A  U
                     GC

ou
c)        A C G A A A C A     G C U A U G U C A
                          C
                    A
                          U
                    A
                          G
                    G
                          A
                    C  GAGU
                    A  U
                    G  C
                    G  C
                    A  G
                     GU

7. Cellules végétales, végétaux, leurs grains ou parties contenant une ou plusieurs des séquences d'ADN selon la revendication 1.

8. Cellules végétales, végétaux, leurs grains ou parties contenant une ou plusieurs des séquences d'ARN selon la revendication 2 ou 3.

9. Tomate, parties de tomate, ses cellules végétales ou grains contenant une ou plusieurs des séquences d'ADN selon la revendication 1.

10. Tomate, parties de tomate, ses cellules végétales ou grains contenant une ou plusieurs séquences d'ARN selon la revendication 2 ou 3.

11. Utilisation de l'ARN ayant une activité de ribozyme selon la revendication 2, pour l'inhibition de la synthèse des enzymes de maturation dans les végétaux.

12. Utilisation selon la revendication 11, caractérisée en ce que les plantes sont des plantes porteuses de fruits.

13. Utilisation selon la revendication 12, caractérisée en ce que les plantes porteuses de fruits sont les tomates.

14. Utilisation selon une ou plusieurs des revendications 11 à 13, caractérisée en ce qu'on utilise en tant que ribozyme la séquence d'ARN selon la revendication 3.

## Revendications (Revendications pour l'(les) Etat(s) contractant(s) suivant(s): ES)

1. Procédé pour la préparation d'un gène ou d'un fragment de gène codant pour un ribozyme avec la séquence d'ADN 
a) 5' CTAGATGATACATGCTGATGAGTCCGTGAGGACGAAACTCCATCTGCA 3'
3'TACTATGTACGACTACTCAGGCACTCCTGCTTTGAGGTAG 5'

b) 5' -CTAGATTAGGTCAGCTGATGAGTCCGTGAGGACGAAACTTGCTACTGCA-3'
3'-TAATCCAGTCGACTACTCAGGCACTCCTGCTTTGAACGATG-5'

c) 5'-CTAGACTGTATCGCTGATGAGTCCGTGAGGACGAAACAAAGCACTGCA-3'
3'-TGACATAGCGACTACTCAGGCACTCCTGCTTTGTTTCGTG-5'
 par synthèse des oligonucléotides, caractérisé en ce qu'on synthétise des oligonucléotides dont les séquences initiale et terminale sont constituées chacune d'au moins 5, de préférence de 7 à 10 nucléotides qui ensemble sont complémentaires à une séquence d'ADN de l'enzyme de maturation végétale à inhiber et qui sont séparées par une séquence d'ADN intercalée entre les deux, qui est constituée en partie de nucléotides spécifiques prédéterminés pour la fonctionnalité du ribozyme et en partie de nucléotides variables.

2. Procédé pour la préparation d'ARN ayant une activité ribozymique de la séquence 
3-KKKKKKKKCA   KKKKKKKK-5
A   C
A    U
G  A   G
C G  G  A
       V
V V
V V
V V V   Boucle
V V V
Ribozyme
 caractérisé en ce qu'on synthétise un oligonucléotide de la séquence 
5'-K'K'K'K'K'K'K'K'CTGAVGAG

3'-K K K K K K K K GACTVCTC

V'V'V'L V'L V'L V'L V'L V'L V'VCGAAACK'K'K'K'K'K'K'-3'
V V V L V L V L V L V L V L V VGCTTTGK K K K K K K -5'
 dans laquelle
K représente les nucléotides A, C, G ou T complémentaires à l'ARN de l'enzyme de maturation végétale,
V représente les nucléotides variables A, C, G ou T, et
V_{L} représente les nucléotides variables A, C, G ou T, le nombre de nucléotides de V_{L} étant un nombre de 0 à 550.
K', V', V_{L}', sont chaque fois les nucléotides A, C, G ou T complémentaires à K, V, V_{L}, lequel nucléotide est cloné dans un vecteur intermédiaire avec un promoteur végétal, puis ensemble avec le promoteur végétal, il est cloné dans un vecteur végétal binaire et une plante est transformée par l'ADN plasmidique ainsi obtenue.

3. Procédé selon la revendication 2, caractérisé en ce qu'on synthétise en tant qu'ARN ayant une activité ribozymique, un ARN de séquence

a)        C U A C C U C A     G U A C A U A G U
                          C
                  A
                          U
                  A
                          G
                          A
                  G
                  C  G^A G^U
                  A  U
                  G  C
                  G  C
                 A   U

b)        A U C G U U C A     G A C U G G A U U
                          C
                  A
                          U
                  A
                          G
                          A
                  G
                  C  G^A G^U
                  A  U
                  G  C
                  G  C
                 A    U
                   G^C

ou
c)        A C G A A A C A     G C U A U G U C A
                          C
                  A
                          U
                  A
                          G
                  G
                          A
                  C  G^A G^U
                  A  U
                  G  C
                  G  C
                 A    G
                   G^U

4. Utilisation d'ARN ayant une activité ribozymique que l'on peut obtenir selon la revendication 2, pour l'inhibition de la synthèse des enzymes de maturation dans les végétaux.

5. Utilisation selon la revendication 4, caractérisée en ce que les plantes sont des plantes porteuses de fruits.

6. Utilisation selon la revendication 5, caractérisée en ce que les plantes porteuses de fruits sont les tomates.

7. Utilisation selon une ou plusieurs des revendications 4 à 6, caractérisée en ce que on utilise en tant que ribozyme la séquence d'ARN que l'on peut obtenir selon la revendication 3.
